# EUROPEAN PATENT SPECIFICATION

(11) **EP 2 079 674 B1**
(45) Date of publication and mention of the grant of the patent: **29.06.2011**
(21) Application number: 07847103.4
(22) Date of filing: 02.11.2007
(51) Int. Cl.: C07C 41/01, C07C 43/205

(54) **PROCESS FOR THE PREPARATION OF TRIFLUOROETHOXYTOLUENES**
VERFAHREN ZUR HERSTELLUNG VON TRIFLUORETHOXYTOLUENEN
PROCÉDÉ DE PRÉPARATION DE TRIFLUOROÉTHOXYTOLUÈNES

(30) Priority: 06.11.2006 EP 06123547
(43) Date of publication of application: 22.07.2009
(73) Proprietor: Grindeks, a joint stock company, Riga 1057 (LV)
(72) Inventor: ZICANE, Daina, 1058 Riga (LV); JAUNBERGS, Janis, 1013 Riga (LV)
(86) International application number: PCT/EP2007/061818
(87) International publication number: WO 2008/055849

(56) References cited:
- EP-A- 1 283 201
- WO-A-01/90062
- WO-A-02/04419
- WO-A-98/47853
- US-A- 3 900 481
- US-A1- 2005 059 825

## Description

### Technical Field

The present invention relates to an novel process for the preparation a substituted 1,4-*bis*(2,2,2-trifluoroethoxy)benzene of the formula (I) 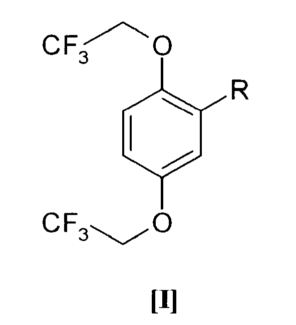 wherein R is methyl
2,5-*bis*(2,2,2-trifluoroethoxy)toluene of the formula [II] is useful as a novel intermediate in the pharmaceutical industry.

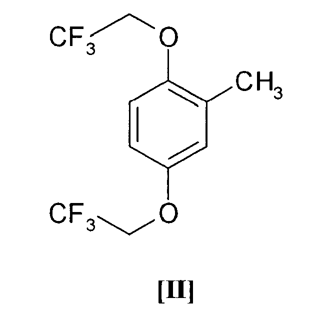

For example use as novel intermediate for the synthesis of the antiarrhythmic drug-Flecainide [III] and pharmaceutically acceptable salts thereof.

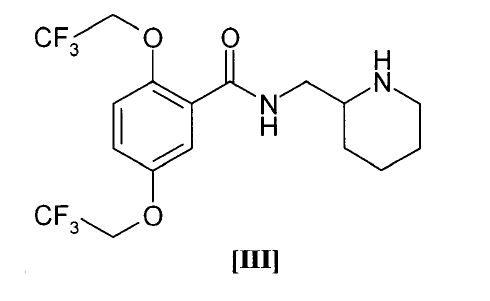

### Background Art

2,5-*bis*(2,2,2-trifluoroethoxy)toluene is a derivative of 1,4-*bis*(2,2,2-trifluoroethoxy)benzene, which as well is used as intermediate for synthesis of Flecainide.

It is known that 1,4-*bis*(2,2,2-trifluoroethoxy)benzene can be obtained by the reaction of 1,4-dibromobenzene with 2,2,2-trifluoroethanol in the presence of sodium hydride, *N,N*-dimethylformamide and cuprous iodide GB 2045760 (RIKER LABORATORIES) 1980.03.18.

A serious disadvantage of using 1,4-dibromobenzene to form 1,4-*bis*(2,2,2-trifluoroethoxy)benzene is that the process requires the reaction of 8 equivalents of 2,2,2-trifluoroethanol while only 2 equivalents are theoretically needed. The use of less than 8 equivalents of 2,2,2-trifluoroethanol results in incomplete conversion to 1,4-*bis*(2,2,2-trifluoroethoxy)benzene, with the starting material and 1-bromo-4-(2,2,2-trifluoroethoxy)benzene as the main impurities. Isolation and purification of desired product from this mixture is not practical on an industrial scale.

Another method involves the process to obtained the 1,4-*bis*(2,2,2-trifluoroethoxy)benzene (III) is by the reaction of 4-fluoro-1-bromobenzene with 2,2,2-trifluoroethanol in the presence of sodium hydride, *N,N*-dimethylformamide and CuBr₂ at about 100-105°C WO 02/066413 (NARCHEM CORPORATION) 2002.02.20.

As disadvantage of this process is that the system NaH with the *N,N-*dimethylformaide has a high safety risk for large-scale industrial synthesis since this system can decompnose exothermically in an uncontrollable manner. BUCKLEY, J.. Report on thermal reaction. Chem. Eng. News. 1982, vol.60, no.28, p.5. , POND, D.. Sodium hydride and DMF. Chem. Eng. News. 1982, vol.60, no.37, p.5,43.

Both of these processes involve three steps to obtain the main intermediate 2,5-*bis*(2,2,2-trifluoroethoxy)benzoic acid, for preparation Flecainide. Disadvantage of multi steps process disclosed in that, what yield of 2,5-*bis*(2,2,2-trifluoroethoxy)benzoic acid is reducing.

In WO 01/90062 A (MERCK PATENT GMBH) 29.11.2001 is disclosed method for the production of trifluoroethoxy-substituted benzoic acids, by reaction of a corresponding halogenated benzoic acid with trifluoroethanol in the solvent tetrahydrofuran, in the presence of a base and a copper salt, It was disclosed one step process for production 2,5-*bis*(2,2,2-trifluoroethoxy)benzoic acid, but 2,5-*bis*(2,2,2-trifluoroethoxy)benzoic acid was obtained in reduced yields

### Disclosure of Invention

The above object is attained by present invention which provides a novel process for the preparation of 2,5-*bis*(2,2,2-trifluoroethoxy)toluene of the formula [II] 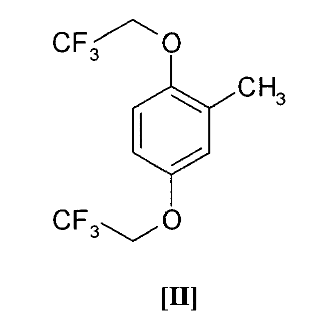 which process comprises reacting a 2,5-dihalotoluene of formula [IV] 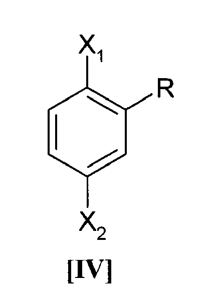 wherein X₁ and X₂ is halogen, R is methyl.

With 2,2,2-trifluoroethanol in the presence alkali metal and a copper containing material.

The process of preparation of 2,5-bis(2,2,2-trifluoroethoxy)toluene by using as starting material 2,5-dihalotolunes has a following advantages:
1. use of metallic sodium instead of sodium hydride and as dipolar aprotic solvent using N,N-dimethylformamide is attempt safer method in industrial production;
2. production of desired final product in high yields;
3. amenability for large scale production which does not require specialized equipment.

The method of the present invention involves the novel initial preparation of 2,5-*bis*(2,2,2-trifluoroethoxy)toluene by reaction 2,5-dihalotoluene with 2,2,2-trifluoroethanol in the presence alkali metal and copper containing material. The 2,2,2-trifluoroathanol reacts in about 2-10 fold molar excess of the 2,5-dihalotoluene to replace the 2,5-dihalotoluene aromatic halogen substituents with trifluoroethoxy groups. Bases that enable the reaction include alkali metals, e.g., metallic sodium. A copper containing material is used as a catalyst in the reaction, e.g., copper (II) sulphate. For performing reaction *N,N*-dimethylformamide can be used as an dipolar aprotic solvent. The best mode for carrying out the reaction is a temperature of about 85 to 105°C.

As it is mentioned above 2,5-*bis*(2,2,2-trifluoroethoxy)toluene can be used as a novel intermediate for preparation Flecainide and pharmaceutically acceptable salts in high yields up to 82%. The preparation of Flecainide can be released by a sequential combination of four process steps, starting from 2,5-dibromotoluene:
1. replacement of the 2,5-dibromotoluene aromatic bromine substituents by trifluoroethoxy groups, and, thus, preparation of 2,5-*bis*(2,2,2-trifluoroethoxy)toluene or any of the intermediate substitution products 2-bromo-5-trifluorethoxytoluene and 5-bromo-2-trifluorethoxytoluene,
2. oxidation of 2,5-*bis*(2,2,2-trifluoroethoxy)toluene with potassium or sodium permanganate, to produce 2,5-*bis*(trifluorethoxy)benzoic acid,
3. activation of 2,5-*bis*(2,2,2-trifluorethoxy)benzoic acid in-situ with a chloroformate, and substitution of the leaving carbonate group with 2-(aminomethyl)pyridine, and
4. hydrogenation of 2,5-*bis*(2,2,2-trifluorethoxy)-N(pyrid-2-yl-methyl)benzamide to obtain Flecainide acetate.

### Best Mode for Carrying Out the Invention

The present invention will be described in more detail with the aid of the following examples.

### Preparation of 2,5-bis(2,2,2-trifluoroethoxy)toluene from 2,5-dibromotoluene

2,2,2-trifluoroethanol (55.0 g, 0.550 mol) was added to dioxane (125 mL) in a glass vessel fitted with a reflux condenser. Sodium metal (11.5 g, 0.500 mol) was added in portions of 2-3 grams to the solution, resulting in a temperature increase from 22°C to 90°C. The solution was stirred at 85-105°C until the sodium dissolution was completed, then *N,N*-dimethylformamide (100 mL) was added, followed by 2,5-dibromotoluene (I) (42.5 g, 0.170 mol) and anhydrous copper (II) sulphate (2.9 g, 0.018 mol). The reaction mixture was stirred at 95-100°C for 4 hours, and then cooled to 25-30°C and poured into 900 ml of a cold (5-10°C) 40% aqueous methanol solution.

Concentrated hydrochloric acid was added (∼25 mL, 0.300 mol), until pH=1-2. The crystallization suspension was stirred for 1 hour at -5 to 0°C, the solid white precipitate was filtered, after which the reaction vessel and the product cake on the filter were rinsed with 50 mL of water. The intermediate 2,5-*bis*(2,2,2-trifluoroethoxy)toluene (II) was dried at ambient temperature and pressure for 5 hours, then at 22 to 24°C and at a reduced pressure for 4 hours. The 2,5-*bis*(2,2,2-trifluoroethoxy)toluene was obtained as a white or off-white powder (45.5 g, 92%) having a melting point of 37°C to 42°C.

### Industrial Application

The 2,5-*bis*(2,2,2-trifluoroethoxy)toluene [II] can be used as intermediate in pharmaceutical industry. For example, can be used as useful intermediate for obtaining Flecainide [III] and pharmaceutically acceptable salts. By using 2,5-*bis*(2,2,2-trifluoroethoxy)toluene as intermediate yield of Flecainide [III] and pharmaceutically acceptable salts can be increased to 86%.

## Claims

1. 2,5-bis(2,2,2-trifluoroethoxy)toluene of the formula (II)

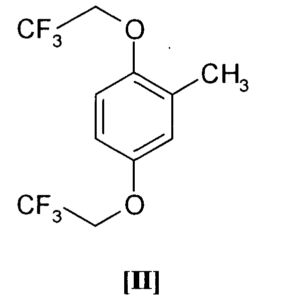

2. The process for the preparation of 2,5-bis(2,2,2-trifluoroethoxy)toluene of the formula [II] 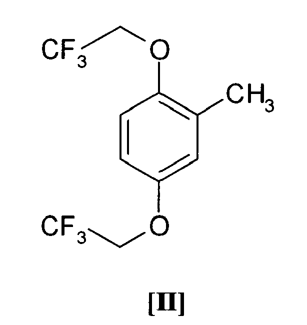 which process comprises reacting a 2,5-dihalotoluene of the formula [IV] 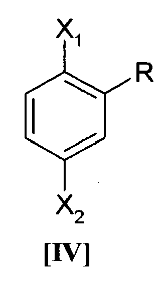 wherein X₁ and X₂ is halogen, R is methyl;
with 2,2,2-trifluoroethanol in the presence of a alkali metal and a copper containing material.

3. The process of claim 2 wherein the halogen is bromide.

4. The process of claim 2 wherein the reaction is carried out in the presence of copper (II) sulphate.

5. The process of claim 2 wherein the alkali metal is metallic sodium.

6. The process of claim 2 wherein the reaction is conducted is an aprotic solvent.

7. The process of claim 6 wherein said aprotic solvent is a dipolar aprotic solvent or an N-containing heterocycle or mixtures thereof.

8. The process of claim 7 wherein the dipolar aprotic solvent is N,N-dimethylformamide.

9. The process of claim 2 wherein the molar ratio, between 2,5-dihalotoluene and 2,2,2-trifluoroethanol is comprised from 1:2 to 1:10, preferably 1:6.

## Patentansprüche

1. Formeln (II) 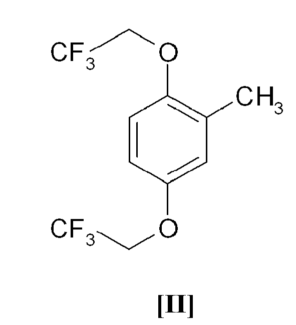 2,5-bis(2,2,2-trifluoroethoxy)-Toluol

2. Verfahren zur Gewinnung der Formel [II] 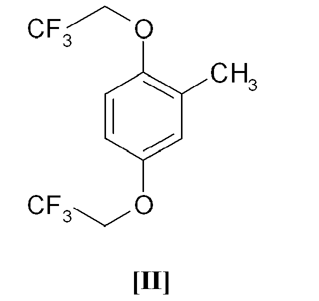 2,5-bis(2,2,2-trifluoroethoxy)-Toluol durch die Reaktion von der Formel [IV] 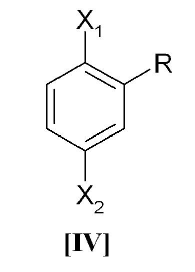 2,5-Dihalotoluol, worin X₁ und X₂ Halogene und R Methyl sind;
mit 2,2,2-Trifluoroethanol in Anwesenheit eines Alkalimetalls oder eines kupferhaltigen Materials.

3. Verfahren nach Anspruch 2, worin ein Bromid das Halogen ist.

4. Verfahren nach Anspruch 2, worin die Reaktion in Anwesenheit von Kupfer(II)-sulfat stattfindet.

5. Verfahren nach Anspruch 2, worin das Natrium-Metall das Alkalimetall ist.

6. Verfahren nach Anspruch 2, worin die Reaktion in aprotischem Lösungsmittel abläuft.

7. Verfahren nach Anspruch 6, worin das obengenannte aprotische Lösungsmittel ein dipolares aprotisches Lösungsmittel oder ein stickstoffhaltiger Heterocyclus oder eine Mischung aus diesen ist.

8. Verfahren nach Anspruch 7, worin ein N,N-Dimethylformamid das dipolare aprotische Lösungsmittel ist.

9. Verfahren nach Anspruch 2, worin das Molverhältnis von 2,5- Dihalotoluol zu 2,2,2-Trifluoroethanol zwischen 1:2 und 1:10 liegt, am besten aber 1:6 ist.

## Revendications

1. Formules (II) 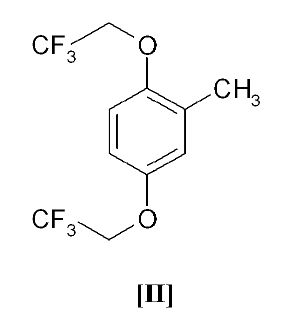 2,5-bis(2,2,2-trifluoroethoxy)toluène

2. Le processus pour la préparation du 2,5-bis(2,2,2-trifluoroethoxy)toluène de formule [II] 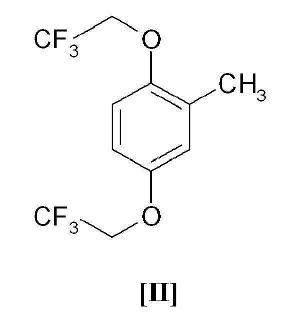 où réagisse le 2,5-dihalo toluène de formule [IV] 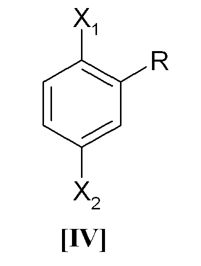 où X₁ et X₂ est halogène, R est méthyle;
avec 2,2,2- trifluoroéthanol en présence du métal alcalin et du matériel, contenant le cuivre.

3. Le processus de la 2^{ième} revendication, où halogène est le bromure.

4. Le processus de la 2^{ième} revendication, où la réaction se passe en présence de sulfate de cuivre (II).

5. Le processus de la 2^{ième} revendication, où le métal alcalin est le sodium métallique.

6. Le processus de la 2^{ième} revendication, où la réaction est faite dans le solvant aprotique.

7. Le processus de la 6^{ième} revendication où le solvant aprotique ci-dessus mentionné et le solvant aprotique dipolaire ou un hétérocycle, contenant l'azote ou leur mélange.

8. Le processus de la 7^{ième} revendication où le solvant aprotique dipolaire est le N,N-dimethylformamide.

9. Le processus de la 2^{ième} revendication, où la proportion molaire entre le 2,5-dihalo toluène et le 2,2,2-trifluoroéthanol est entre 1:2 jusqu'au 1:10 ou mieux - 1:6.
